# EUROPEAN PATENT APPLICATION

(11) **EP 1 920 700 A1**
(43) Date of publication of application: **14.05.2008**
(21) Application number: 06023580.1
(22) Date of filing: 13.11.2006
(51) Int. Cl.: A61B 1/00

(54) **Seal element**

(71) Applicant: Pentax Europe GmbH, 22527 Hamburg (DE)
(72) Inventor: Janell, Daniela, 22609 Hamburg (DE); Heath, Andy, Abbeymead, Gloucester GL4 5TZ (GB)
(74) Representative: Rohnke, Christian

(57) **Abstract**

What is claimed is a seal element for sealing the insertion of a cylindrically shaped generally flush surfaced first body inserted into an opening provided in a second body, particularly for sealing the insertion of an endoscopic accessory inserted into the instrument opening of an endoscope, the seal element comprising a through-passage for entering a first body, the through-passage being surrounded at least partially by a flexible seal material for sealingly closing the through-passage around an inserted first body, and the seal element further comprising a first form-fitting means being
adopted to fit to a counterpart form-fitting means, particularly a stud, particularly a Luer stud, particularly provided at the opening of a second body, and being
oriented aligned with the through-passage, particularly such that the through-passage is oriented aligned with the opening when the first form-fitting means of the seal element is form-fittingly mounted to the counterpart form-fitting means at the opening, and
comprising a sealing means to seal any gap between the first form-fitting means and a counterpart form-fitting means when form-fittingly mounted,

**characterized in that**
the flexible seal material surrounding the through-passage is formed as a flexible rim protruding radially inwards and towards a puncture.

## Description

The present invention relates to a seal element and a method of producing a seal element, wherein the seal element is provided for sealing the insertion of a cylindrically shaped generally flush surfaced first body inserted into an opening provided in a second body. "Insertion" means any site between the first body and the second body, particularly where fluid tight sealing of the space, particularly the gap, between the first body and the second body is possible at all. It is well preferred that the seal element according to the invention provides for fluid tight sealing not only when the first body is inserted into the second body, but also at the procedure of entering, removing or any other relative motion between the first body and the second body. Additionally to a truly flush surface, "generally flush" can be understood for example to be a surface provided by wires tightly bent to form an overall cylindrical shape - or any other surface forming a fluid tight seal in contact with an elastomeric rim or lip like that one being element of the seal element according to the invention. Particularly, the seal element is provided for sealing the insertion of an endoscopic accessory inserted into the instrument opening of an endoscope. An endoscopic accessory can be a biopsy forceps, an aspiration needle, a snare, a stent, or a catheter or even a cholangioscope. The seal element comprises a through-passage for entering a first body, the through-passage being surrounded at least partially by a flexible seal material for sealingly closing the through-passage around an inserted first body. The seal element further comprises a first form-fitting means being adopted to fit a counterpart form-fitting means, particularly a stud, particularly a Luer stud, particularly provided at the opening of a second body. The first form-fitting means furthermore being oriented aligned with the through-passage particularly such that the through-passage is oriented aligned with the opening when the first form-fitting means of the seal element is form-fittingly mounted to the counterpart form-fitting means at the opening. The first form-fitting means furthermore comprising a sealing means to seal any gap between the first form-fitting means and a counterpart form-fitting means when form-fittingly mounted.

It is a widespread technical problem to seal the insertion of a cylindrically shaped generally flush surfaced first body inserted into an opening provided in a second body. For example, at endoscopical surgery, the endoscope comprises a tubular device inserted into the human body. The tip of the tubular device is located where surgery is to be performed. The surgeon can enter different endoscopic accessories into the tubular device - and in particular into the instrument opening at the other end of the tubular device in order to guide the instrument via the tubular device with the instrument tip beyond the tip of the tubular device. This way, the tip of the endoscopic accessory can be precisely located where surgery is to be performed. If the surgery requires a different instrument, the surgeon pulls the instrument out of the tubular device and out of the instrument opening in order to enter the required different instrument.

Here like in other technical situations, a fluid pressure is effective from inside the opening. In the particular example of endoscopy, air is pumped into the human body in order to cause a certain cavity wherein the endoscopical surgery can be performed. It is understandable that this pressure causes blood and secretion to reach into the tubular device and finally to drip out of the instrument opening unless hindered by a reliable seal.

In this technical example as well as in other similar technical situations, the function of the seal is reliably required even if the cylindrical shaped first body, in particular the endoscopic accessory is axially and/or rotationally moved within the opening and within the seal - even to a huge extent for example at changing the instrument.

Consequently it is a problem - being solved by the present invention - to provide for a seal element of the above mentioned kind having improved sealing properties even at motion of the cylindrically shaped first body inserted into the opening provided in the second body.

This problem is solved according to the invention by a seal element having the features of claim 1, 7 or 10 or by a method of producing a seal element according to claim 11.

Preferred embodiments are enclosed in the dependant claims and in the description and drawings.

It is one aspect of the present invention to provide seal element for sealing the insertion of a cylindrically shaped generally flush surfaced first body inserted into an opening provided in a second body, particularly for sealing the insertion of an endoscopic accessory inserted into the instrument opening of an endoscope, the seal element comprising a through-passage for entering a first body, the through-passage being surrounded at least partially by a flexible seal material for sealingly closing the through-passage around an inserted first body, and the seal element further comprising a first form-fitting means being adopted to fit to a counterpart form-fitting means, particularly a stud, particularly a Luer stud, particularly provided at the opening of a second body, and being oriented aligned with the through-passage, particularly such that the through-passage is oriented aligned with the opening when the first form-fitting means of the seal element is form-fittingly mounted to the counterpart form-fitting means at the opening, and comprising a sealing means to seal any gap between the first form-fitting means and a counterpart form-fitting means when form-fittingly mounted.

According to the invention, the flexible seal material of the seal element is formed as a flexible rim protruding radially inwards and toward a generally straight lined puncture.

The puncture according to the invention, e.g. a penetration of the flexible seal material by a needle or any other acute tip, is closed elastically because of the elastic property of the flexible seal material - in other words, the puncture does not have a macroscopic diameter. Known seal elements for similar technical purposes are provided with a through-hole of a certain diameter. Other known seal elements are provided with a membrane which is penetrated at first use by the first body inserted into the opening. In both cases the missing cylindrical shape of the through-hole results in worse performance of the seal as the contact surface between the limitation of the through-hole of the seal element and the cylindrically shaped generally flush surfaced first body is very little extended. In contrast, according to the invention, the puncture provide for a cylindrical contact surface not only extending circularly around the inserted second body, in particular the inserted endoscopic accessory but also for an axial extension of the sealing contact surface. Therefore, the puncture is to be understood as a straight penetration of the flexible seal material, for example performed by a sharp needle.

It is well preferred that the rim comprises an annular edge protruding into a main direction radially inwards and axially away from the sealing surface and having an inner diameter being larger than the diameter of the puncture. Still the inner diameter of the annular edge is adopted to be smaller than the outer diameter of a first body, whereby the inserted first body displaces the flexible rim, when for example the tip of the first body contacts the annular edge and presses the annular edge including the rim axially.

This causes the puncture to be displaced axially and consequently to be opened elastically to a certain extent, which is adopted to be wider than the outer diameter of the inserted first body so that the sealing surface generally does not contact the inserted first body protecting the sealing surface from friction and wear.

This preferred annular edge preferably is rounded of rather than sharp-edged in order to easier glide over the generally flush surfaced first body when inserted.

Furthermore, the preferred annular edge is provided axially on both sides of the sealing surface at the rim such that the at least two annular edges protrude axially away from the sealing surface into the two different axial directions.

Advantageously, this provides for the described positive effect of protecting the sealing surface from friction and wear as well when the first body is inserted into the opening as when the first body is removed and thus moved axially into the other direction.

In order to support entering the first body into the seal element, the rim is taped inwards to the direction in which the first body is to be inserted. This provides advantageously for an easy unhindered motion at entering the first body into the opening.

Furthermore it is well preferred that the rim is formed on a part being separate from a body of the sealing element and being mounted into the body of the seal element under radial compression of the separate part. Advantageously, this causes radial compressive preload in the puncture , which provides for further enhanced surface contact between the sealing surface and the inserted first body - and thus for improved sealing properties.

Under a different aspect of the present invention, the above mentioned problem is solved by a seal element of the mentioned kind, namely for sealing the insertion of a cylindrically shaped generally flush surfaced first body inserted into an opening provided in a second body, particularly for sealing the insertion of an endoscopic accessory inserted into the instrument opening of an endoscope, the seal element comprising a through-passage for entering a first body, the through-passage being surrounded at least partially by a flexible seal material for sealingly closing the through-passage around an inserted first body, and the seal element further comprising a first form-fitting means being adopted to fit to a counterpart form-fitting means, particularly a stud, particularly a Luer stud, particularly provided at the opening of a second body, and being oriented aligned with the through-passage, particularly such that the through-passage is oriented aligned with the opening when the first form-fitting means of the seal element is form-fittingly mounted to the counterpart form-fitting means at the opening, and comprising a sealing means to seal any gap between the first form-fitting means and a counterpart form-fitting means when form-fittingly mounted.

Under this aspect, the seal element according to the invention comprises at least two flexible rims protruding radially inwards and towards a through-hole or a predetermined penetration point, preferably towards a puncture or a circular cylindrical through-hole. These at least two flexible rims are axially spaced apart from each other defining a reservoir space in between the two rims. Consequently, any body fluid like blood or secretion having passed one of the two flexible rims is housed in the reservoir space and thus kept there instead of further pouring out of the seal.

Preferably an outer wall of the reservoir space of the seal element is translucent enough to make visible non-transparent liquid like secretion, blood or other body fluids inside the reservoir space. Advantageously, this is indicating prior use of the sealing element and provides for information to the physician or endoscopy assistance personnel that this seal element should soon be replaced (if multi-usable), or should not be used again (if designed to be for single-use) - in order to provide for reliable sealing properties.

According to a further aspect of the present invention, the above mentioned problem is solved by a seal element of the above mentioned kind, namely for sealing the insertion of a cylindrically shaped generally flush surfaced first body inserted into an opening provided in a second body, particularly for sealing the insertion of an endoscopic accessory inserted into the instrument opening of an endoscope, the seal element comprising a through-passage for entering a first body, the through-passage being surrounded at least partially by a flexible seal material for sealingly closing the through-passage around an inserted first body, and the seal element further comprising a first form-fitting means being adopted to fit to a counterpart form-fitting means, particularly a stud, particularly a Luer stud, particularly provided at the opening of a second body, and being oriented aligned with the through-passage, particularly such that the through-passage is oriented aligned with the opening when the first form-fitting means of the seal element is form-fittingly mounted to the counterpart form-fitting means at the opening, and comprising a sealing means to seal any gap between the first form-fitting means and a counterpart form-fitting means when form-fittingly mounted.

This seal element comprises a flexible area between the first form-fitting means and the flexible seal material having the following elastical properties: The flexible area is easier deformed elastically than the first form-fitting means is dismounted from a counterpart form-fitting means. In other words and for example at removal of a first body from the seal element, forces acting upon the flexible seal material elastically deform the flexible area - instead of dismounting the first form-fitting means from a counterpart form-fitting means. Advantageously, any axial and/or radial motion of the inserted first body relative to the seal will result only in elastical deformation of the flexible area instead of disadvantageously resulting in an unwanted dismounting of the seal element from the opening. This aspect of the present invention may be understood as an elastical buffer against motion of the inserted first body and against the otherwise resulting unwanted removal of the seal element from its form-fitting means provided at the opening - consequently improving reliability of the sealing properties.

It is a further aspect of the present invention to provide a method for producing a seal element of the mentioned kind, namely for sealing the insertion of a cylindrically shaped generally flush surfaced first body inserted into an opening provided in a second body, particularly for sealing the insertion of an endoscopic accessory inserted into the instrument opening of an endoscope, the seal element comprising a through-passage for entering a first body, the through-passage being surrounded at least partially by a flexible seal material for sealingly closing the through-passage around an inserted first body, and the seal element further comprising a first form-fitting means being adopted to fit to a counterpart form-fitting means, particularly a stud, particularly a Luer stud, particularly provided at the opening of a second body, and being oriented aligned with the through-passage, particularly such that the through-passage is oriented aligned with the opening when the first form-fitting means of the seal element is form-fittingly mounted to the counterpart form-fitting means at the opening, and comprising a sealing means to seal any gap between the first form-fitting means and a counterpart form-fitting means when form-fittingly mounted.

According to this aspect of the invention the seal element is produced by molding the seal element comprising the flexible seal material molded as a flexible membrane extending radially through the through-passage before performing the step of puncturing the membrane. Consequently, a sealing opening of the seal element is not produced at the molding step, thus resulting in a production of the precise straight lined sealing opening, which may for example be produced by penetrating the membrane with a tip of a very sharp needle.

These and other features and advantages will become apparent from the following description of the attached drawing.

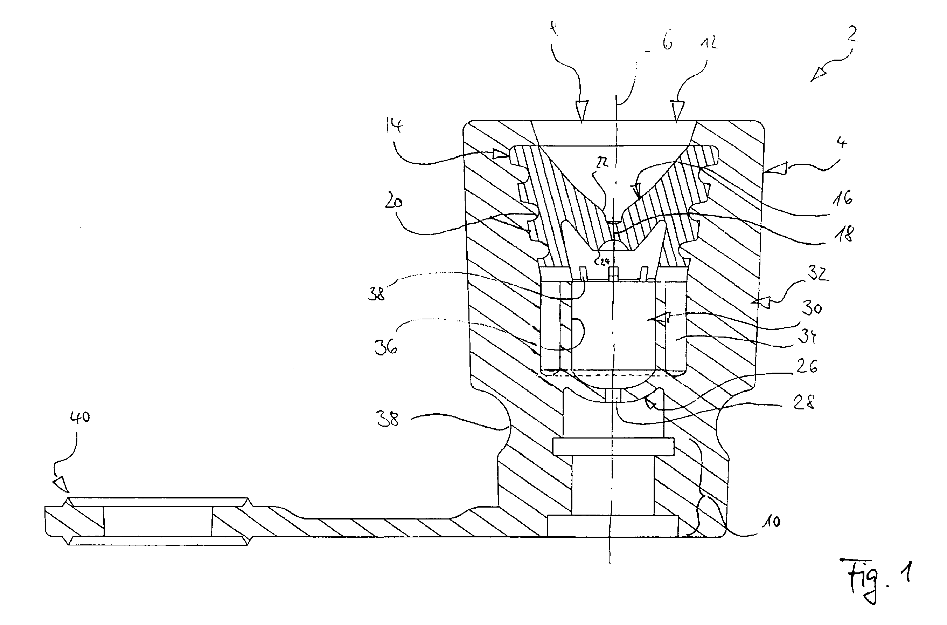

- **Figure 1**: shows a side-sectional view of an embodiment of the present invention.

According to figure **1** a seal element **2** is provided having a molded body **4** of a resilient material like Silicone or Thermo Plastic Elastomer (TPE), particularly medical grade TPE.

The body **4** is rotationally symmetrical around an axis **6.** A through-passage **8** is extending through the body **4** of the seal element **2** along the axis **6.** At the lower end of the rotationally symmetrical through-passage **8,** there is a form-fitting means **10** adopted to fit to a counterpart form-fitting means, namely a Luer stud. The form-fitting means **10** is the negative form of a standardized Luer stud - thus providing for mounting the seal element **2** to a Luer stud by being slipped over the Luer stud elastically. From the alignment of the form-fitting means, a first body, for example an endoscopic accessory (not shown), can be inserted through the above opening **12** of the through-passage **8** and further on into the Luer stud, which may lead into a second body (not shown), for example into the instrument opening of an endoscope.

In order to provide for safe sealing of the insertion of such a first body into such Luer stud, the seal element **2** provides several features.

The resilient material of the seal element **2** provides for safe form-fit of the form-fitting means **10** around the Luer stud and for safe sealing at this location. When a cylindrically shaped generally flush surfaced first body (not shown) is inserted into the through-passage of the seal element **2,** a separate part **14** made of flexible seal material sealingly surrounds the inserted first body (not shown) at the upper end of the through-passage **8.** The part **14** comprises an annular sealing lip or rim **16** protruding radially inwards and towards a puncture **18.** The puncture **18** is aligned with the center axis **6.** The rim **16** is tapered inwards into the direction (in figure 1 downwards) in which the first body (not shown) is to be inserted. The part **14** is mounted into the body **4** of the seal element **2** under radial compression of the separate part. This causes radial compressive preload in the puncture **18.** For this purpose, the outer profile **20** of the part **14** has a slightly larger outer diameter than the according inner profile **20** of the body **4** in which the part **14** is form-fittingly mounted.

The rim **16** furthermore comprises two annular edges **22, 24** protruding radially inwards and axially away from the sealing surface **18.** The inner diameter of the annular edges **22, 24** is smaller than the outer diameter of a first body (not shown) to be inserted into the seal element. Consequently, an inserted first body displaces the flexible rim **16** upon contact with the annular edge **22** axially and such that the puncture **18** opens like a mouth and wider than the outer diameter of the inserted first body. This causes the sealing surface **18** not to contact the inserted first body (not shown) - thus protecting the sealing surface **18** from friction and wear particularly at entering the first body.

The same effect is caused by the annular edge **24** at pulling out a priorly inserted first body (not shown).

The annular edges **22, 24** are rounded off in order to better slide over the generally flush surfaced first body (not shown).

In figure 1 further down the way of the through-passage **8,** there is a second flexible rim **26** having a circular cylindrical through-hole **28.** The first rim **16** and the second rim **26** are axially spaced apart from each other defining a reservoir space **30** in between the two rims **16, 26** for housing body fluid (not shown) potentially having passed the second rim **26.** The cylindrical outer wall **32** of the reservoir space **30** is translucent enough to make visible such non-transparent liquid, particularly body fluid like secretion or blood, inside the space - thus indicating prior use of the sealing element.

The reservoir space **30** comprises an annular reservoir **34.** The annular reservoir space **34** surrounds the through-passage **8** and is separated from the through-passage **8** by a generally tubular inner wall **36** and is in fluid-connection with the through-passage **8** via eight conduits **38.**

At the lower end, the outer generally cylindrical profile of seal element **2** comprises a waist **38** providing for a flexible area between the form-fitting means **10** and the sealing rims **16, 26.** In the area of the tail **38,** the flexible area is easier deformed elastically than the form-fitting means **10** is dismounted from the Luer stud (not shown). In other words at removal of a first body from the seal element, forces acting upon the sealing rims **16, 26** elastically deform the flexible area **38 -** instead of dismounting the form-fitting means **10** from the Luer stud.

The seal element comprises a tether **40** which can be mounted form-fittingly for example to a stud (not shown) close to the above mentioned Luer stud (not shown) in order to hold the seal element **2** generally in place for later re-mounting when dismounted from the Luer stud.

## Claims

1. Seal element
for sealing the insertion of a cylindrically shaped generally flush surfaced first body inserted into an opening provided in a second body, particularly for sealing the insertion of an endoscopic accessory inserted into the instrument opening of an endoscope, the seal element
comprising a through-passage for entering a first body, the through-passage being surrounded at least partially by a flexible seal material for sealingly closing the through-passage around an inserted first body, and the seal element further
comprising a first form-fitting means being
adopted to fit to a counterpart form-fitting means, particularly a stud, particularly a Luer stud, particularly provided at the opening of a second body, and being
oriented aligned with the through-passage, particularly such that the through-passage is oriented aligned with the opening when the first form-fitting means of the seal element is form-fittingly mounted to the counterpart form-fitting means at the opening, and
comprising a sealing means to seal any gap between the first form-fitting means and a counterpart form-fitting means when form-fittingly mounted,
**characterized in that**
the flexible seal material surrounding the through-passage is formed as a flexible rim protruding radially inwards and towards a puncture.

2. The sealing element according to claim 1, **characterized in that** the rim comprises an annular edge protruding into a main direction radially inwards and axially away from the sealing surface and having an inner diameter being larger than the diameter of the puncture, whereby an inserted first body displaces the flexible rim upon contact with the annular edge such that the puncture opens generally wider than the outer diameter of the inserted first body and thus the sealing surface generally does not contact the inserted first body protecting the sealing surface from friction and wear.

3. The sealing element according to one of the preceding claims, **characterized in that** the annular edge is rounded off rather than sharp-edged.

4. The sealing element according to one of the preceding claims, **characterized in that** the rim comprises annular edges axially on both sides of the sealing surface, wherein the at least two annular edges protrude axially away from the sealing surface into the two different directions.

5. The sealing element according to one of the preceding claims, **characterized in that** the rim is tapered inwards into the direction in which the first body is to be inserted.

6. The sealing element according to one of the preceding claims, **characterized in that** the rim is formed on a separate part being mounted into the seal element under radial compression of the separate part causing radial compressive preload in the puncture.

7. Seal element, particularly according to one of the preceding claims,
for sealing the insertion of a cylindrically shaped generally flush surfaced first body inserted into an opening provided in a second body, particularly for sealing the insertion of an endoscopic accessory inserted into the instrument opening of an endoscope, the seal element
comprising a through-passage for entering a first body, the through-passage being surrounded at least partially by a flexible seal material for sealingly closing the through-passage around an inserted first body, and the seal element further
comprising a first form-fitting means being
adopted to fit to a counterpart form-fitting means, particularly a stud, particularly a Luer stud, particularly provided at the opening of a second body, and being
oriented aligned with the through-passage, particularly such that the through-passage is oriented aligned with the opening when the first form-fitting means of the seal element is form-fittingly mounted to the counterpart form-fitting means at the opening, and
comprising a sealing means to seal any gap between the first form-fitting means and a counterpart form-fitting means when form-fittingly mounted,
**characterized in that**
the flexible seal material surrounding the through-passage is formed as at least two flexible rims protruding radially inwards and towards a puncture or a through-hole or a predetermined penetration point, preferably towards a puncture, and being axially spaced apart from each other defining a reservoir space inbetween the two rims for housing body fluid having passed one of the two flexible rims.

8. The sealing element according to the preceding claim, **characterized in** having an outer wall translucent enough to make visible non-transparent liquid, particularly body fluid like secretion or blood, inside the space thus indicating prior use of the sealing element.

9. The sealing element according to one of the two preceding claims, **characterized by** an annular reservoir space surrounding the through-passage and being separated from the through-passage by a generally tubular inner wall and being in fluid-connection with the through-passage via at least one conduit.

10. Seal element, particularly according to one of the preceding claims,
for sealing the insertion of a cylindrically shaped generally flush surfaced first body inserted into an opening provided in a second body, particularly for sealing the insertion of an endoscopic accessory inserted into the instrument opening of an endoscope, the seal element
comprising a through-passage for entering a first body, the through-passage being surrounded at least partially by a flexible seal material for sealingly closing the through-passage around an inserted first body, and the seal element further
comprising a first form-fitting means being
adopted to fit to a counterpart form-fitting means, particularly a stud, particularly a Luer stud, particularly provided at the opening of a second body, and being
oriented aligned with the through-passage, particularly such that the through-passage is oriented aligned with the opening when the first form-fitting means of the seal element is form-fittingly mounted to the counterpart form-fitting means at the opening, and
comprising a sealing means to seal any gap between the first form-fitting means and a counterpart form-fitting means when form-fittingly mounted,
**characterized by**
a flexible area between the first form-fitting means and the flexible seal material, the flexible area being easier elastically deformed than the first form-fitting means is dismounted from a counterpart form-fitting means, such that forces applied to the flexible seal material - for example at removal of a first body from the seal element - elastically deform the flexible area instead of dismounting the first form-fitting means from a counterpart form-fitting means.

11. Method of producing a seal element, particularly according to one of the preceding claims
for sealing the insertion of a cylindrically shaped generally flush surfaced first body inserted into an opening provided in a second body, particularly for sealing the insertion of an endoscopic accessory inserted into the instrument opening of an endoscope, the seal element
comprising a through-passage for entering a first body, the through-passage being surrounded at least partially by a flexible seal material for sealingly closing the through-passage around an inserted first body, and the seal element further
comprising a first form-fitting means being
adopted to fit to a counterpart form-fitting means, particularly a stud, particularly a Luer stud, particularly provided at the opening of a second body, and being
oriented aligned with the through-passage, particularly such that the through-passage is oriented aligned with the opening when the first form-fitting means of the seal element is form-fittingly mounted to the counterpart form-fitting means at the opening, and
comprising a sealing means to seal any gap between the first form-fitting means and a counterpart form-fitting means when form-fittingly mounted,
**characterized by** the steps:
a) Molding the seal element, the flexible seal material being formed as a flexible membrane extending radially through the through-passage, and
b) Puncturing the membrane.
